# DEMANDE DE BREVET EUROPEEN

(11) **EP 3 530 306 A1**
(43) Date de publication de la demande: **28.08.2019**
(21) Numéro de dépôt: 19165755.0
(22) Date de dépôt: 25.01.2016
(51) Int. Cl.: A61M 5/32, A61M 5/31, A61M 5/00

(54) **PROCÉDÉ DE FABRICATION DE SERINGUES PRÉ-REMPLIES À AIGUILLE COLLÉE**

(30) Priorité: 26.01.2015 FR 1550575; 02.06.2015 FR 1554990
(62) Demande divisionnaire de: 16701360.6
(71) Demandeur: Biocorp Production, 63500 Issoire (FR)
(72) Inventeur: ANEAS, Antoine, 63200 MENETROL (FR)
(74) Mandataire: Lavoix

(57) **Abrégé**

L'invention concerne un procédé de fabrication de seringues pré-remplies à aiguille collée, comprenant des étapes consistant à monter un dispositif (D) de protection de l'aiguille sur des corps de seringue (2), , à disposer les corps de seringue (2), équipés du dispositif de protection, dans des logements (O204) prévus dans un support (200), à placer le support (200) dans un container (100) pour le transport, à sortir le support du container, à remplir chaque corps de seringue avec un principe actif. Le support (200) est un support standard. En outre le procédé comprend des étapes consistant à insérer un piston dans chaque seringue, à sortir les seringues (1) de leur support pour l'inspection et l'étiquetage et à emballer individuellement chaque seringue avec un emballage primaire et un emballage secondaire.

## Description

L'invention concerne un procédé de fabrication de seringues pré-remplies à aiguille collée.

Actuellement, les seringues pré-remplies avec aiguille collée sont fabriquées de la manière suivante : Le verrier fabriquant des corps de seringue en verre assemble un protège-aiguille souple ou rigide à l'extrémité de chaque corps de seringue avec aiguille collée. Les corps de seringue, alors équipés chacun d'un protège-aiguille, sont ensuite disposés dans un support en matière plastique, appelé « rack ». Ce rack est en fait une plaque délimitant une série de trous de réception des corps de seringue. Les racks sont ensuite disposés dans des containers de transport, qui sont operculés par un film en matériau synthétique. Les containers sont stérilisés à l'oxyde d'éthylène, qui est apte à traverser l'opercule. Lorsque les containers sont réceptionnés par le laboratoire pharmaceutique, ils sont ouverts dans une ambiance stérile et les racks sont sortis et placés sur des machines automatiques de remplissage. Chaque corps de seringue est alors rempli d'un principe actif, puis un piston, aussi appelé «joint de piston », est enfoncé à l'intérieur de chaque corps de seringue. Les corps de seringue sont alors sortis des racks pour les placer sur une ligne d'inspection et d'étiquetage. Une fois cette opération effectuée, les corps de seringue sont déplacés sur une autre ligne d'assemblage pour y incorporer le système de sécurité et une tige de poussée du piston, qui est apte à activer le système de sécurité une fois que l'injection est terminée.

L'inconvénient majeur de ce procédé est qu'il est nécessaire d'investir dans des infrastructures volumineuses et couteuses pour doter les seringues d'un système de sécurité après usage. Un autre inconvénient est que le système de sécurité choisi est particulièrement encombrant, de manière que les seringues ne peuvent être emballées ni avec un emballage primaire (blister) standard, ni avec un emballage secondaire (boite en carton) standard. En particulier, l'emballage final est particulièrement encombrant, ce qui entraine un coût de transport élevé. Enfin, la tige de poussée du piston utilisée n'est pas standard puisqu'elle comporte des rondelles pour activer le système de sécurité une fois l'injection terminée.

WO-A-2016/115477 divulgue un procédé de fabrication de seringues dans lequel un support spécifique doit être utilisé, ce qui rend le procédé peu intéressant sur le plan industriel. En outre, le support n'est pas adapté à des corps de seringues de faible diamètre.

C'est à ces inconvénients qu'entend plus particulièrement remédier l'invention en proposant un procédé de fabrication de seringues pré-remplies qui peut être mis en oeuvre avec une installation moins volumineuse et moins couteuse.

A cet effet l'invention concerne un procédé de fabrication de seringues pré-remplies à aiguille collée, qui comprend des étapes consistant à :
a) monter un dispositif de protection de l'aiguille sur des corps de seringue, le dispositif comprenant un système de sécurité après usage et un protège-aiguille,
b) disposer les corps de seringue, équipés du dispositif de protection, dans des logements prévus dans un support,
c) placer le support dans un container standard, fermer le container et stériliser le container pour le transport,
d) dans une ambiance stérile, sortir le support du container.
Selon l'invention, le support est un support standard, alors que le procédé comprend, en outre, des étapes consistant à :
e) remplir chaque corps de seringue avec un principe actif,
f) insérer un piston dans chaque seringue,
g) sortir les seringues de leur support pour l'inspection, l'assemblage d'une tige de piston et l'étiquetage, et à
h) emballer individuellement chaque seringue avec un emballage primaire et un emballage secondaire.

Grâce à ce procédé, le dispositif de protection de l'aiguille peut être mis en place directement par le verrier, c'est-à-dire en même temps que le protège-aiguille, du fait que ce dernier est intégré au dispositif. Les laboratoires pharmaceutiques n'ont donc pas besoin de prévoir une ligne d'assemblage dédiée au montage du système de sécurité après remplissage. Cela permet de gagner de l'espace dans les infrastructures d'assemblage des seringues. En outre, le dispositif de protection intégrant le protège-aiguille est suffisamment compact, pour que les seringues peuvent être disposées, à l'étape b), avec leur dispositif de protection dans un support, ou « rack » standard. De même, des tiges de piston standard peuvent être utilisées. L'emballage final est aussi standard et peu encombrant. Ainsi, le procédé industriel de fabrication existant n'est pas perturbé, mais au contraire simplifié.

Selon des aspects avantageux mais non obligatoires de l'invention, le procédé de fabrication peut incorporer une ou plusieurs des caractéristiques suivantes, prises dans toute combinaison techniquement admissible :
- L'étape a) est effectuée par un verrier fabricant des corps de seringue.
- A l'étape c), le support est placé dans le container de manière que les seringues ne touchent pas le fond du container.
- Le dispositif monté sur chaque corps de seringue présente à son extrémité distale un chanfrein annulaire qui coopère sans accrocs avec un logement correspondant du support lors des étapes b) et g).
- À l'étape g), la tige de piston est vissée sur le piston.
- L'emballage primaire utilisé à l'étape g) est un emballage coque transparent.
- L'emballage secondaire utilisé à l'étape g) est une boite en carton.
- Chaque dispositif comprend des moyens de fixation élastique sur un corps de seringue correspondant, qui se fixent, lors de l'étape a), par rapprochement avec le corps de seringue.
- Les moyens de fixation incluent des pattes élastiques qui coopèrent, lors de l'étape a), avec une partie d'extrémité du corps de seringue, cette partie d'extrémité étant conformée pour bloquer le dégagement des pattes une fois le dispositif monté.
- Les étapes d) à f) sont automatisées.
- Les logements du support standard ont un diamètre de 9,3 mm

L'invention et d'autres avantages de celle-ci apparaitront plus clairement à la lumière de la description qui va suivre d'un mode de réalisation d'un dispositif de protection d'une aiguille et d'un procédé de fabrication conforme à leur principe, faite uniquement à titre d'exemple et en référence aux dessins annexés dans lesquels :
- les figures 1, 3, 5, 7, 9, 11 et 13 représentent chacune une vue de côté d'une seringue à aiguille collée, comprenant un dispositif de protection d'une aiguille,
- les figures 2, 4, 6, 8, 10, 12 et 14 représentent chacune une coupe longitudinale, à plus grande échelle, de la seringue correspondant aux figures mentionnées ci-dessus,
- les figures 15 et 16 sont des coupes longitudinales et à plus grande échelle, du dispositif de protection de la seringue des figures 1 à 14, dans deux plans de coupe différents,
- les figures 17 à 21 sont des coupes longitudinales représentant des étapes d'assemblage du dispositif des figures 1 à 16 sur une seringue à aiguille collée,
- la figure 22 est une vue en perspective représentant un container de transport pour la fabrication de seringues à aiguilles collées telle que représentée à la figure 1,
- la figure 23 est une coupe selon le plan XXIII de la figure 22,
- la figure 24 est une vue à plus grande échelle de l'encerclé XXIV de la figure 23, et
- les figures 25 à 27 sont des vues en perspective représentant chacune une étape du procédé de fabrication conforme à l'invention.

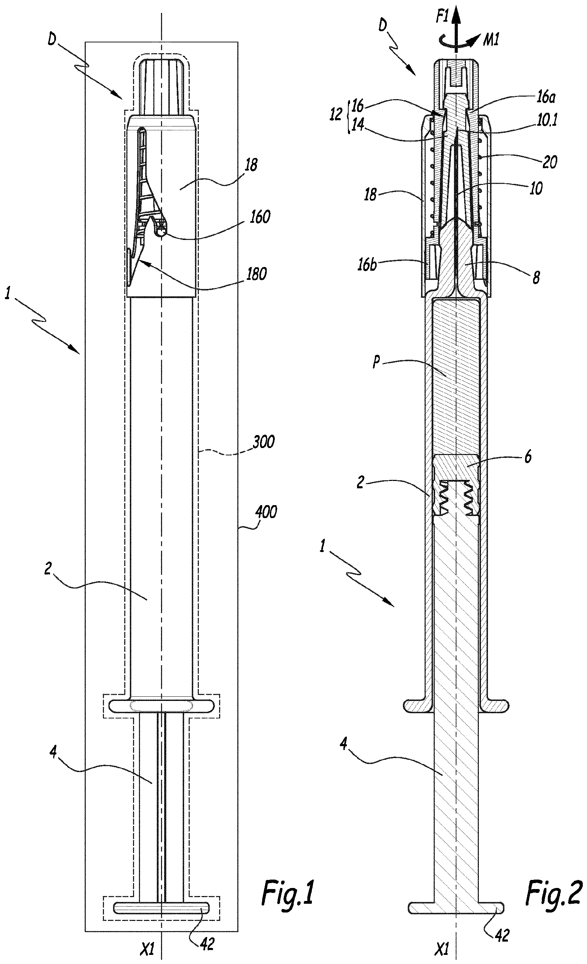

Sur chacune des figures 1 à 14 est représentée une seringue à aiguille collée 1. Cette seringue 1 est du type pré-remplie et s'étend selon un axe longitudinal X1. Elle comprend un corps de seringue 2, généralement en verre, qui est globalement tubulaire et centré sur l'axe X1. Le corps 2 comporte un nez 8 dans lequel est enfoncée une aiguille creuse 10. L'aiguille 10 est fixée à l'intérieur du nez 8 par collage. Le nez 8 présente un décrochement externe 80. L'aiguille 10 comporte une extrémité distale 10.1 taillée en biseau. Le corps de seringue 2 renferme un principe actif P, tel qu'un médicament. La seringue 1 comporte également une tige standard 4 qui est équipée à son extrémité d'un joint 6. Le joint 6 sert de piston pour éjecter le principe actif P à travers l'aiguille creuse 10, c'est pourquoi le joint 6 est couramment appelé « piston » ou « joint de piston ». Le joint 6 est attaché à la tige 4, c'est-à-dire qu'il est lié en translation avec la tige 4 dans les deux sens de déplacement. Plus précisément, la tige 4 est vissée à l'intérieur du joint 6. A l'opposé du joint 6 selon l'axe X1, la tige 4 est équipée d'une palette 42 sur laquelle l'utilisateur peut exercer un effort de poussée vers le nez 8. La tige 4 est déplaçable en translation par rapport au corps 2 selon l'axe X1, c'est-à-dire qu'elle est apte à coulisser à l'intérieur du corps de seringue 2.

Dans cette description, la direction avant ou distale désigne une direction parallèle à l'axe longitudinal X1 et tournée vers l'épiderme du patient dans des conditions normales d'utilisation de la seringue 1, alors que la direction arrière ou proximale est orientée à l'opposé par rapport à la zone d'injection, du côté de la palette 42.

La seringue 1 comporte, à l'avant, un dispositif D de protection de l'aiguille 10. Ce dispositif D est adapté pour être monté sur l'extrémité avant du corps de seringue. Le dispositif D intègre un protège-aiguille 12 qui permet, d'une part, de conserver l'aiguille 10 propre avant l'utilisation de la seringue 1 et éviter ainsi la pollution du principe actif P, et d'autre part, de protéger l'aiguille 10 contre toute action mécanique extérieure. Par exemple, le protège-aiguille 12 empêche l'aiguille 10 de se tordre ou de se briser avant usage.

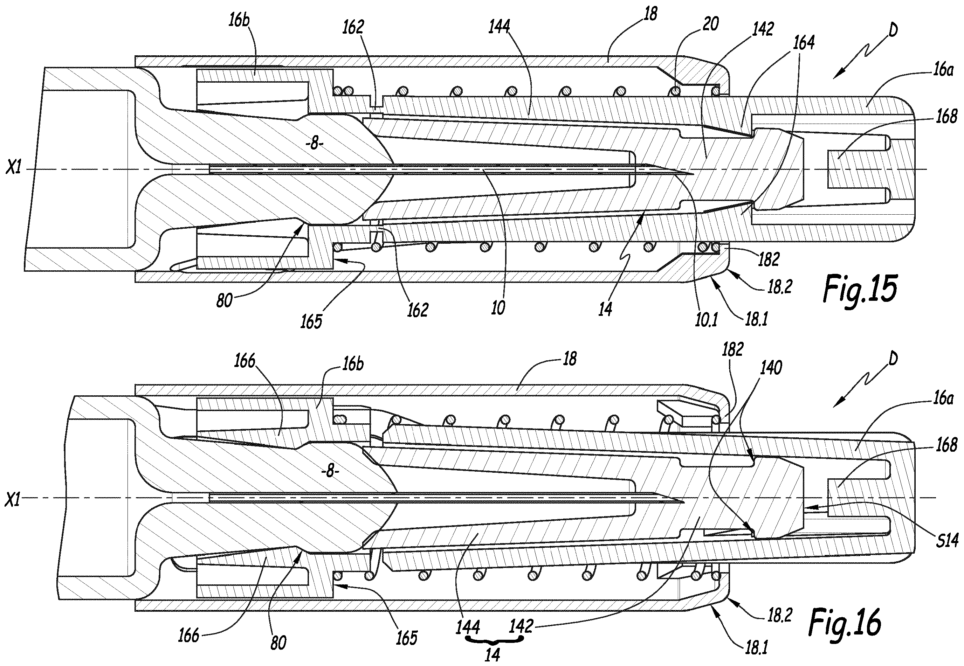

Le protège-aiguille 12 est un protège-aiguille rigide comprenant un embout souple 14 dans lequel est enfoncée l'aiguille 10 et une gaine rigide 16 qui enveloppe l'embout 14. L'embout 14 est en élastomère (caoutchouc) ou en matière thermoplastique injectable, alors que la gaine rigide 16 est en matière plastique, dans l'exemple en polyéthylène à haute densité (PEHD). Le protège-aiguille 12 est mieux visible aux figures 15 et 16. Comme visible sur ces figures, l'embout 14 comporte, à l'avant, une section rétrécie 142 formant un épaulement annulaire 140 qui élargit le diamètre de l'embout 14 en allant vers l'avant. L'embout 14 comporte également, à l'arrière, une jupe 144 entourant une partie de l'aiguille 10. L'embout 14 est comprimé contre le nez 8 du corps de seringue 2. La jupe 144 est tronconique et s'élargit vers l'arrière de l'embout 14.

La gaine rigide 16 est en deux parties 16a et 16b qui sont détachables l'une de l'autre par un mouvement de rotation relatif entre les deux parties 16a et 16b. En effet, les deux parties de la gaine 16a et 16b sont reliées entre elles par des pontets sécables 162, conçus pour être rompus lors de l'application d'un moment M1 de rotation relative entre les deux parties de la gaine 16a et 16b. La partie 16a est disposée à l'avant de la partie 16b. Les parties 16a et 16b sont chacune de forme tubulaire centrée sur l'axe longitudinal X1.

La partie arrière 16b de la gaine 16 est encliquetée autour du nez 8 de la seringue 1, c'est-à-dire qu'elle comprend des moyens de fixation élastique autour du nez 8 de la seringue 1. Ces moyens de fixation comprennent des pattes élastiques 166 qui sont conformées pour venir se coincer dans le décrochement 80 du nez 8. La partie 16b est donc solidaire en rotation du corps de seringue 2. La partie arrière 16b de la gaine rigide 16 comporte aussi deux pions diamétralement opposés 160, qui font saillie radialement vers l'extérieur par rapport à l'axe longitudinal X1. Un seul de ces pions est cependant visible sur les figures.

Le dispositif D comprend des moyens de liaison en translation, le long de l'axe X1, entre l'embout 14 et la partie 16a de la gaine 16. Ces moyens de liaison comprennent des dents 164 ménagées sur une surface radiale interne de la partie avant 16a de la gaine. Ces dents 164 font saillie radialement par rapport à l'axe longitudinal X1 vers l'intérieur de la gaine et coopèrent avec le rebord annulaire 140 de l'embout 14, de manière que l'embout 10 est lié en translation avec la partie avant 16a de la gaine 16. Cependant, une rotation de la gaine 16 autour de son axe n'entraine pas de rotation de l'embout 14, c'est-à-dire que la gaine 16 et l'embout 14 ne sont pas solidaires en rotation autour de l'axe longitudinal X1. La partie avant 16a de la gaine 16 comporte, à son extrémité avant, un ergot central 168 s'étendant axialement vers l'arrière. Il existe un jeu axial J2 d'environ 1 mm entre l'ergot 168 et une surface d'extrémité avant S14 de l'embout 14. Le jeu J2 est mesuré parallèlement à l'axe X1. Par ailleurs, il y a un jeu entre la surface extérieure de l'embout 14 et la surface intérieure de la gaine 16. Ce jeu est mesuré selon une direction normale à la surface extérieure de l'embout 14, qui est légèrement oblique par rapport à l'axe X1. Ainsi, l'embout 14 ne risque pas de tourner solidairement à la partie avant 16a de la gaine 16 par frottement.

Le dispositif de protection D comporte également un système de sécurité visant à protéger l'aiguille 10 après usage de la seringue 1, c'est-à-dire lorsque la seringue 1 est retirée du corps du patient. Ce système comprend un manchon extérieur 18, qui est disposé coaxialement autour de la gaine rigide 16. Le manchon 18 est fabriqué en matériau opaque, pour cacher complètement l'aiguille 10. Ce manchon 18 délimite un rebord radial interne 182 au niveau de son extrémité avant et deux évidements 180 dans lesquels les pions 160 sont respectivement insérés. Dans l'exemple, les pions 160 ne dépassent pas des évidements 160 vers l'extérieur. Les évidements 180 servent de guide aux pions 160. Chaque évidement 180 est globalement en forme de Y asymétrique, avec les branches du Y qui s'étendent vers l'arrière. Les branches du Y sont référencées 180a et 180c, alors que sa portion centrale est référencée 180b. Cette portion centrale 180b est une portion droite, c'est-à-dire un couloir. Le dispositif D comprend également des moyens de verrouillage du manchon 18 en position avancée, qui sont activés en fin d'injection. Dans l'exemple, ces moyens de verrouillage sont formés par un logement 180d qui s'étend, à partir de la branche 180c, vers l'avant.

Le manchon extérieur 18 est mobile axialement, c'est-à-dire le long de l'axe X1, entre une position avancée, où il est recouvre l'aiguille 10 et une position reculée, où l'aiguille 10 est découverte. Le système de sécurité comprend des moyens de rappel élastique du manchon extérieur 18 en position avancée. Ces moyens de rappel comprennent un ressort hélicoïdal 20 qui est intercalé entre le rebord radial interne 182 du manchon 18 et un épaulement 165 formé sur la partie arrière 16b de la gaine rigide 16. Le ressort hélicoïdal 20 présente un pas à droite, c'est-à-dire que le sens d'enroulement du ressort 20 est à droite. Cela signifie que le ressort 20 est enroulé à droite, ou dans le sens horaire, lorsque l'on regarde le ressort 20 par le bas aux figures 1 à 14, c'est-à-dire du côté arrière.

Il existe un jeu mécanique radial entre le manchon extérieur 18 et la gaine rigide 16, de sorte que le manchon 18 peut coulisser autour de la gaine 16 sans frottements. En revanche, Il n'existe pas ou peu de jeu mécanique radial entre le manchon 18 et la surface extérieure du corps de seringue 2, de sorte que le dispositif D n'est pas très encombrant radialement. En particulier, l'épaisseur du manchon 18 est choisie pour que les seringues 1 puissent être insérées dans les trous d'un support standard.

Par ailleurs, le manchon 18 comporte à son extrémité distale un chanfrein annulaire 18.1 qui converge par rapport à un axe central du manchon 18 vers l'avant et qui est relié au rebord radial interne 182 par un congé périphérique 18.2.Ci-dessous sont décrites différentes étapes d'utilisation de la seringue 1 en référence aux figures 1 à 14.

Tout d'abord, l'utilisateur doit retirer le protège-aiguille rigide 12 pour pouvoir réaliser l'injection. Pour ce faire, il applique le moment M1 autour de l'axe X1, comme représenté à la figure 2, pour tourner la partie avant 16a par rapport à la partie arrière 16b et rompre les pontets 162. Une fois les pontets 162 rompus, l'utilisateur peut retirer la partie avant 16a de la gaine rigide 16, comme représenté par la flèche F1 à la figure 2. Le retrait de la partie 16a entraine solidairement le retrait de l'embout 14 par coopération des dents 164 avec le rebord annulaire 140 de l'embout 14. Ainsi, l'embout 14 et la partie avant 16a de la gaine 16 sont retirés de la seringue 1 sans faire tourner l'embout 14 autour de l'aiguille 10, de manière que l'extrémité distale 10.1 de l'aiguille 10, taillée en biseau, ne forme pas de copeaux de matière susceptibles de pénétrer dans l'aiguille 10.

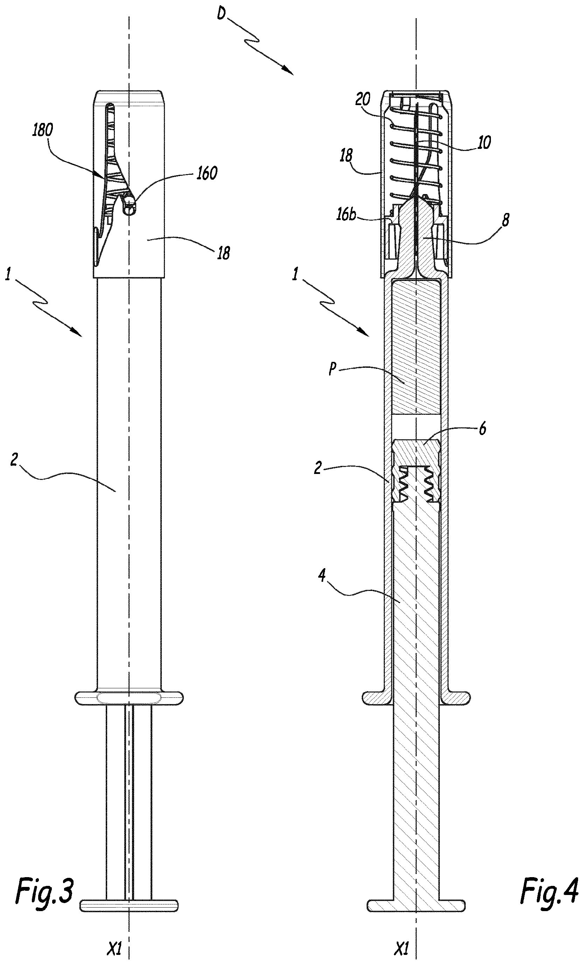

Le retrait de l'embout 14 et de la partie avant 16a de la gaine 16 amène la seringue 1 dans la configuration des figures 3 et 4. Dans cette configuration, l'aiguille 10 est entièrement recouverte par le manchon 18. Tant que la seringue 1 n'a pas été utilisée, les pions 160 de la partie arrière 16b de la gaine 16 sont logés dans la branche 180a des évidements 180.

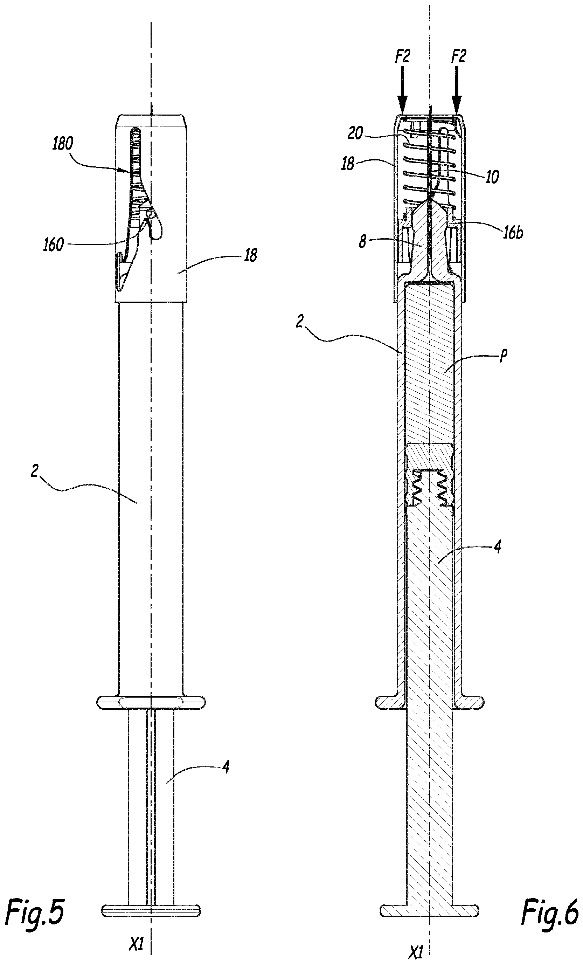

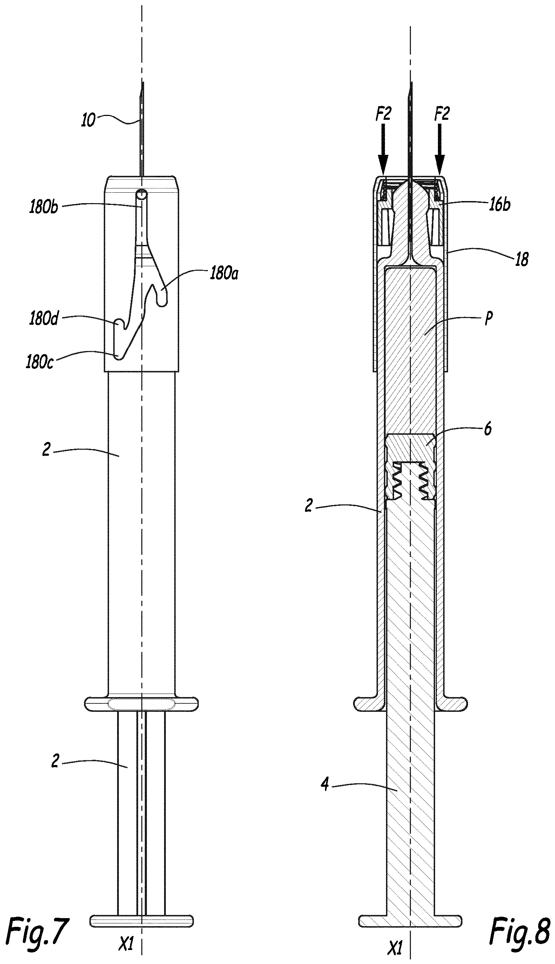

En référence aux figures 5 et 6, lorsque la seringue 1 est amenée contre l'épiderme d'un patient, la pression exercée par le manchon 18 sur la peau entraine le recul du manchon 18, comme représenté par les flèches F2 à la figure 6. Le ressort 20 est alors comprimé, l'aiguille 10 pénètre dans l'épiderme et les pions 160 se déplacent de la branche 180a jusque dans la portion centrale 180b. Le manchon 18 recule autour du corps de seringue 2. Ainsi, l'aiguille 10 n'est pas découverte tant que la seringue 1 n'est pas amenée contre l'épiderme du patient, à la différence des matériels selon WO-A-2013/134465 et WO-A-2007/077463, où l'aiguille est en partie découverte avant que la seringue ne soit amenée contre le corps du patient. Autrement dit, le manchon 18 n'est pas reculé préalablement à l'injection pour laisser apparaitre l'aiguille 10. Ainsi, il n'y a pas de risque de piqûre accidentelle avant l'injection. La poursuite du mouvement amène le manchon extérieur 18 vers sa position reculée, dans laquelle il ne recouvre plus l'aiguille 10. La poursuite du mouvement s'effectue jusqu'à ce que les pions 160 arrivent au fond du couloir 180b des évidements 180, comme représenté aux figures 7 et 8.

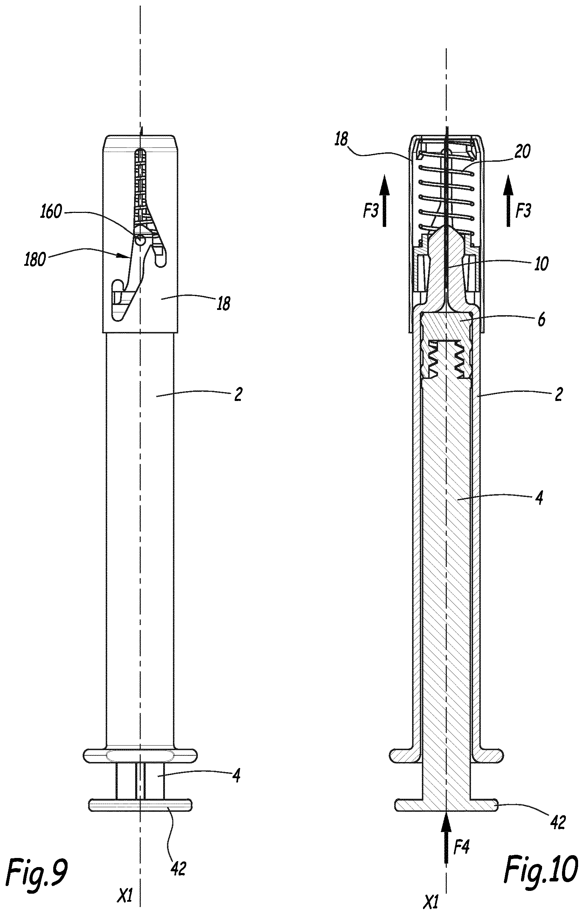

Dans la configuration des figures 7 et 8, l'aiguille 10 de la seringue 1 est complètement enfoncée dans l'épiderme du patient. L'utilisateur peut alors appuyer sur la palette 42 de la tige 4 pour éjecter le principe actif P contenu à l'intérieur de la seringue 1 dans le corps du patient, comme représenté par la flèche F4 à la figure 10.

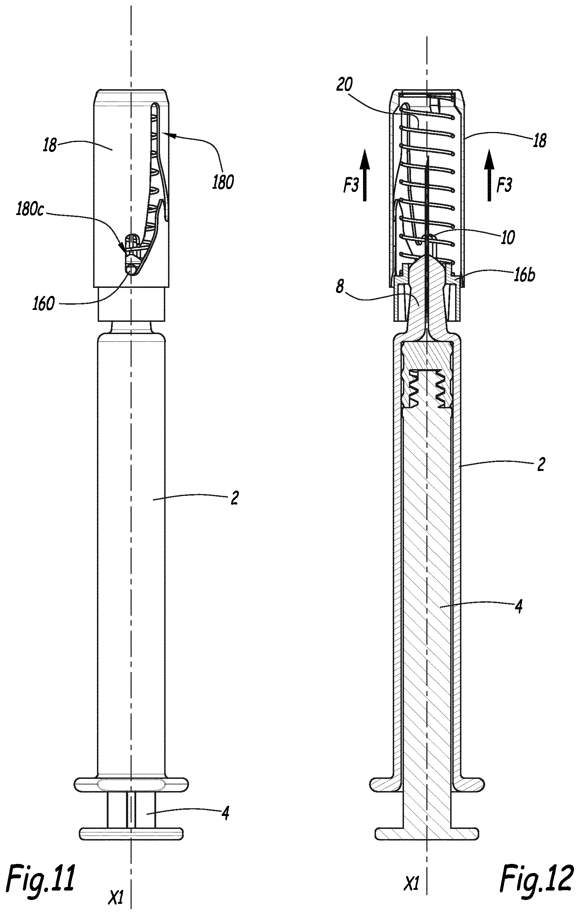

Lorsque l'utilisateur retire la seringue 1 du corps du patient, le manchon extérieur 18 est rappelé élastiquement en position avancée par le ressort 20, comme représenté par les flèches F3 à la figure 10. Le manchon extérieur 18 revient alors en recouvrement de l'aiguille 10 et les pions 160 coulissent dans le couloir 180b des évidements 180 en direction de la branche 180c. La seringue 1 se trouve alors dans la configuration des figures 11 et 12, qui correspond à une configuration de fin d'injection.

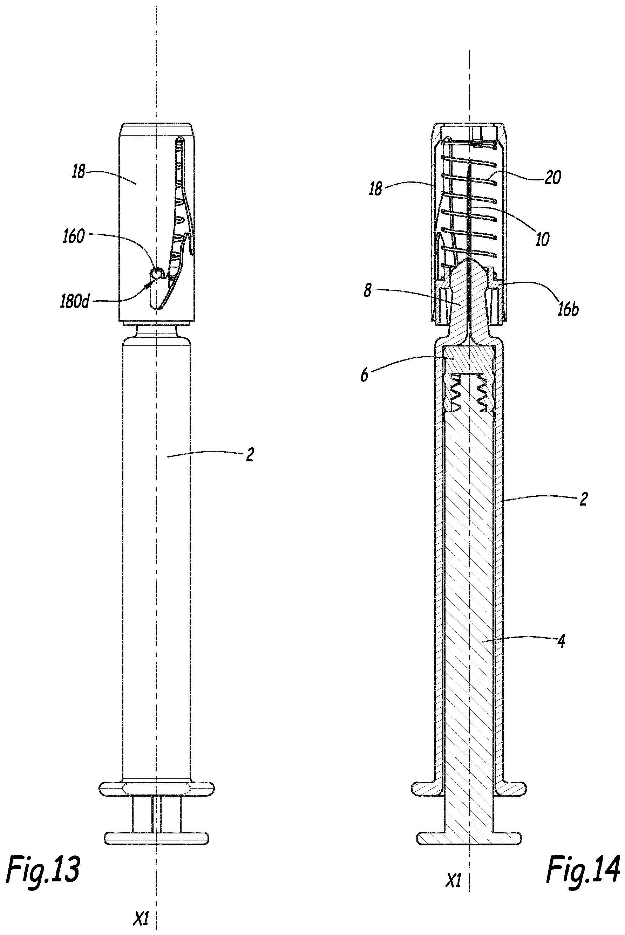

Si, après usage de la seringue 1, un utilisateur maladroit appuie sur le manchon 18, c'est-à-dire tente de reculer le manchon 18, les pions 160 se déplacent alors dans le logement 180d des évidements 180 et le déplacement du manchon 18 vers l'arrière est bloqué, comme représenté sur les figures 13 et 14. Cela constitue une sécurité supplémentaire puisque l'aiguille 10 ne peut plus être découverte en fin d'injection. Plus précisément, le déplacement des pions 160 de la branche 180c vers le logement 180d est favorisé du fait que le ressort 20 a un sens d'enroulement à droite. En effet, ce ressort 20 exerce, lorsqu'il est comprimé, un couple sur le manchon 18 qui est dirigé, du fait de son sens d'enroulement, dans le sens antihoraire en vue de dessus à la figure 11, c'est-à-dire lorsque l'on regarde la seringue 1 du côté de l'aiguille 10. Ce couple permet d'éviter que les pions 160 retournent en direction du couloir 160b si l'utilisateur tente de reculer le manchon 18 après l'injection. Ce couple permet également de guider correctement les pions 160 dans le couloir 180b jusqu'à la branche 180c des évidements 180.

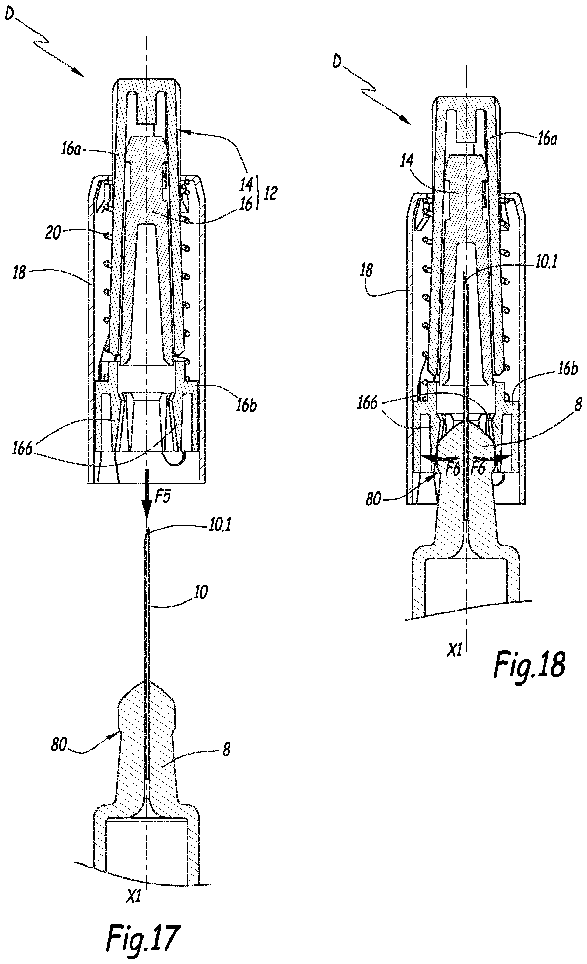

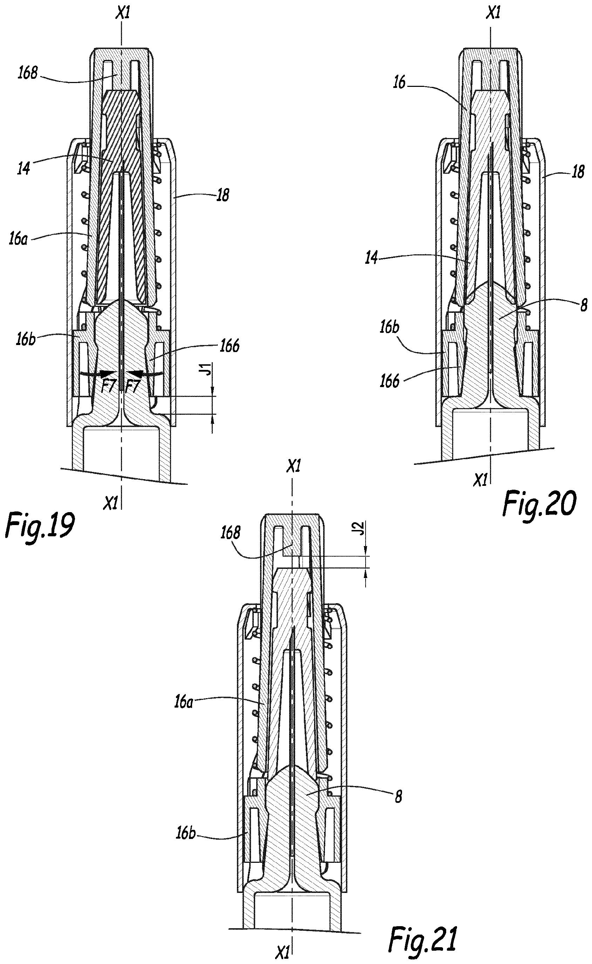

Sur les figures 17 à 21 sont représentées les étapes de montage du dispositif de protection D sur le nez 8 du corps de seringue 2. Une première étape du montage du dispositif de protection D consiste à rapprocher le dispositif D du nez 8, comme représenté par la flèche F5 à la figure 17. En poursuivant le mouvement d'approche dans le sens de la flèche F5 les pattes élastiques 166 sont alors déformées selon une direction radiale centrifuge F6 au contact du nez 8, comme visible à la figure 18. Une fois que les pattes 166 ont dépassé le décrochement 80 du nez 8, elles viennent s'encliqueter contre ce dernier par retour élastique de la matière, comme représenté par les flèches F7 à la figure 19. Le nez 8 de la seringue 1 est conformé pour bloquer le dégagement des pattes 166. Il existe un jeu J1 d'environ 1,5 mm entre l'extrémité libre des pattes 166 et le corps de seringue 2. Le jeu J1 est mesuré parallèlement à l'axe X1.

Lors d'une dernière étape illustrée par les figures 19 à 21, on appuie sur le protège-aiguille 12 de manière à bien enfoncer l'aiguille 10 à l'intérieur de l'embout 14. Comme visible à la figure 20, l'embout 14 se retrouve alors comprimé axialement, entre l'ergot 168 de la gaine rigide 16 et le nez 8 du corps de seringue 2 et le jeu J1 entre l'extrémité libre des pattes 166 et le corps de seringue 2 est rattrapé. Lorsque l'on relâche la pression sur le protège-aiguille 12, la matière de l'embout 14 reprend sa forme initiale et repousse la gaine 16 vers l'avant. La gaine 16 revient alors en position d'encliquetage et un jeu axial J2 existe entre la surface d'extrémité avant S14 de l'embout 14 et l'ergot 168.

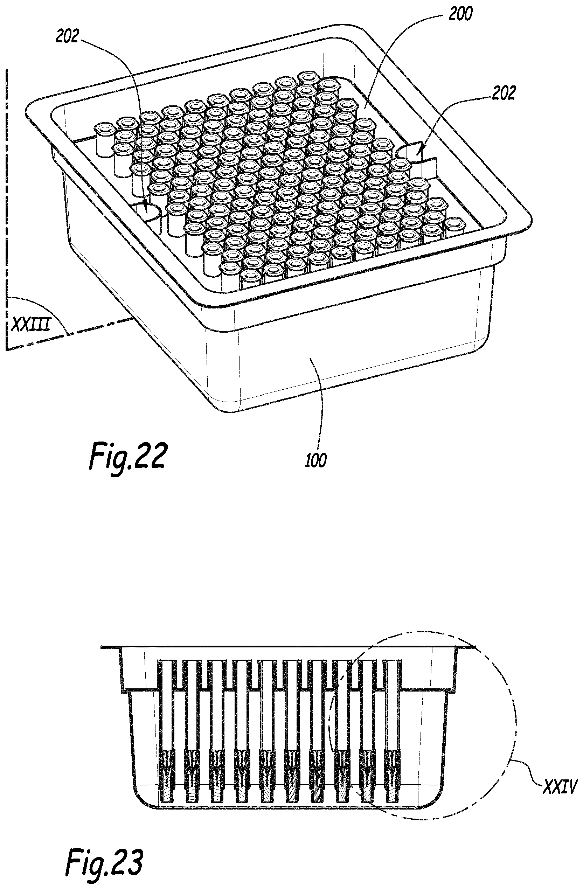

Ci-dessous est décrit, en référence aux figures 22 à 27, un procédé de fabrication de seringues pré-remplies à aiguille collée 1, telles que décrites précédemment. Dans le présent document, une pièce est considérée comme « standard » si elle se trouve déjà dans le commerce, c'est-à-dire si elle est déjà utilisée dans les procédés de fabrication existants.

Le procédé comprend une première étape a) consistant à monter un dispositif de protection D sur chaque seringue à aiguille collée 1. Cette première étape a) est effectuée par un verrier fabricant des corps de seringue 2. Comme le dispositif de protection D intègre le protège-aiguille 12, le système de sécurité et le protège-aiguille 12 sont montés d'un seul bloc sur le corps de seringue 2. Cela permet de simplifier le procédé d'assemblage existant. En outre, grâce aux moyens de fixation élastique, le montage du dispositif D sur un corps de seringue 2 s'effectue simplement par rapprochement des deux éléments. Une deuxième étape b) consiste à disposer les seringues 1, chacune équipée d'un dispositif de protection D, dans des logements O204 prévus dans un support standard 200. Ce support 200 est couramment appelé « rack » et consiste en une plaque rectangulaire en matière plastique, qui est trouée. Les trous O204 de la plaque 200 forment les logements de réception des seringues 1. Les trous O204 ont un diamètre inférieur au diamètre d'une surface d'enveloppe extérieure du dispositif D. De manière standard, ces trous O204 ont un diamètre de 9,3 mm. Une surface d'enveloppe extérieure du dispositif D présente un diamètre extérieur qui est donc inférieur à 9,3 mm, dans l'exemple de l'ordre de 9 mm. Les trous O204 sont délimités par des colonnettes 204 qui font saillie vers le haut perpendiculairement à la plaque 200. Le support 200 délimite seize rangées de dix trous O204.

Grâce à la présence du chanfrein 18.1 et du congé 18.2 prévus à son extrémité distale, chaque dispositif D est inséré sans accroc dans un logement O204 du support 200. Autrement dit, lorsque les seringues sont insérées dans le support 200, la surface extérieure du manchon 18 glisse contre la paroi du trou correspondant O204.

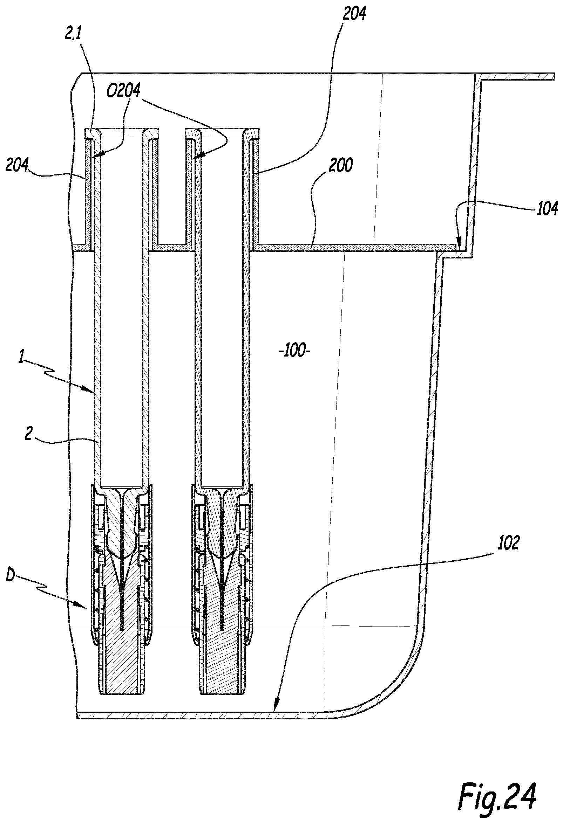

Le corps 2 de chaque seringue comporte, à une extrémité opposée au nez 8, un épaulement annulaire 2.1 prévu pour venir en appui contre l'extrémité libre d'une colonnette 204, de manière à empêcher le corps de seringue 2 de tomber sous l'effet de la gravité. La plaque 200 comporte deux poignées 202 facilitant sa préhension. Lorsque toutes les seringues sont disposées dans le rack 200, celui-ci est positionné, lors d'une troisième étape c), à l'intérieur d'un container standard 100. Le container 100 comprend à cet effet deux épaulements opposés 104, qui s'étendent chacun sur la totalité d'un côté du container 100. Comme visible aux figures 23 et 24, les seringues 1 ne touchent pas le fond 102 du container 100 lorsque le rack 200 est placé dans le container 100.

Le container 100 est ensuite operculé de manière étanche par un film en matériau synthétique non représenté, qui est non-étanche aux gaz. Le container 100 est rendu stérile par injection de gaz, notamment de l'oxyde d'éthylène. L'oxyde d'éthylène pénètre à travers l'opercule du container, ce qui permet de stériliser les seringues à l'intérieur du container. A l'issue de l'étape c), les containers 100 sont envoyés par le verrier au laboratoire pharmaceutique. Autrement dit, le procédé comprend une étape postérieure à l'étape c) consistant à transporter les containers 100 d'un point à un autre, en l'occurrence du verrier au laboratoire pharmaceutique.

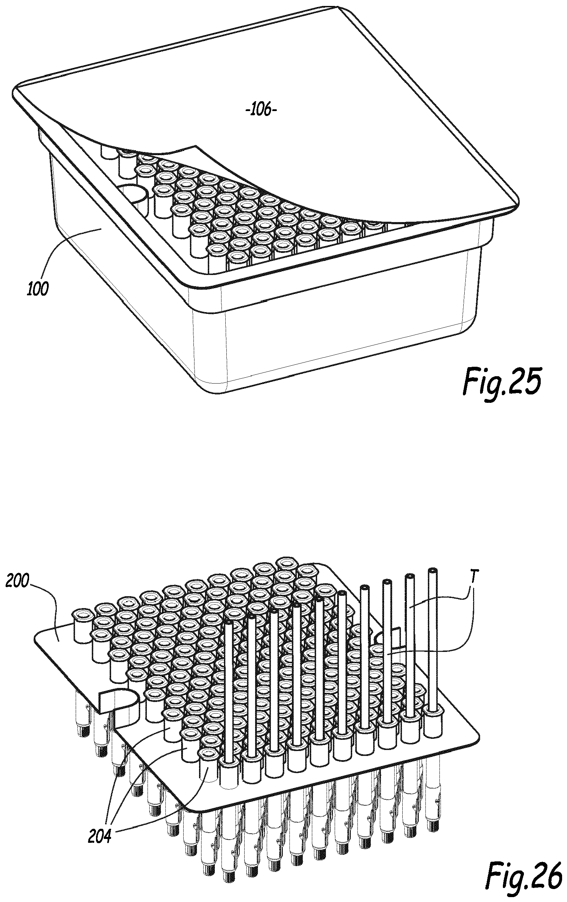

Comme visible à la figure 25, les containers 100 sont ouverts, lors d'une quatrième étape d), chez le laboratoire pharmaceutique dans une ambiance stérile et les racks 200 sont sortis de manière automatisée des containers 100. Lors d'une cinquième étape e), les racks 200 sont placés sur des machines automatiques de remplissage non-représentées pour remplir les corps de seringue 2 avec un principe actif P, comme un médicament. Comme visible à la figure 26, la machine de remplissage comprend une rangée de dix tubes de remplissage T, adaptés pour être inséré chacun dans un corps de seringue 2. Le principe actif est alors injecté à travers les tubes T. Une fois que tous les corps de seringue d'une rangée sont remplis, les tubes T sont sortis hors des corps 2 et le support 200 est déplacé pour remplir une autre rangée de seringues.

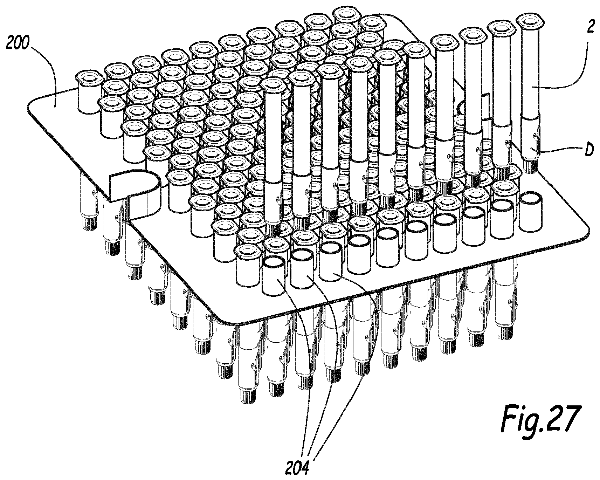

Une fois toutes les seringues 1 remplies, un piston, ou joint de piston 6 est inséré lors d'une sixième étape f) à l'intérieur de chaque corps de seringue 2. Comme visible à la figure 27, les corps 2 des seringues 1, alors remplis de principe actif, sont sortis, lors d'une septième étape g), des racks 200. L'extraction des seringues hors du support 200 s'effectue sans accrocs du fait de la présence du chanfrein annulaire 18.1 et du congé 18.2 sur chaque dispositif D. Autrement dit, lorsque les seringues sont extraites du support 200, la surface extérieure du manchon 18 glisse contre la paroi du trou correspondant O204. Les seringues 1 sont ensuite placées sur une ligne pour l'inspection, l'assemblage de la tige de piston 4 et l'étiquetage. Précisément, la tige 4 est vissée dans le joint 6.

Lors d'une huitième étape h), les seringues pré-remplies 1 sont emballées individuellement avec un emballage primaire 300 et un emballage secondaire 400. L'emballage primaire 300 est un emballage coque transparent standard, mieux connu sous le nom de « blister ». L'emballage secondaire 400 est une boite en carton standard. Le produit emballé est compact et donc facilement transportable. Ces emballages sont représentés, respectivement en traits pointillés et en traits pleins à la figure 1 uniquement.

Grâce à ce nouveau procédé, les seringues 1 sont livrées au laboratoire pharmaceutique avec leur dispositif de protection D intégré, si bien que le laboratoire pharmaceutique n'a pas à assembler le dispositif de protection sur chaque seringue. Le laboratoire pharmaceutique n'a donc pas besoin de disposer d'une ligne d'assemblage dédiée au montage des dispositifs de protection sur les seringues, ce qui permet d'économiser de la place. De plus, le dispositif de protection D est très compact, de sorte que la seringue 1 peut être disposée dans un trou du rack directement avec le dispositif de protection.

En variante non représentée, des moyens de rappel différents d'un ressort peuvent être envisagés pour rappeler le manchon extérieur 18 en position avancée en fin d'injection.

En variante non représentée, un seul évidement 180 est pratiqué dans le manchon 18. De même, le manchon 18 peut délimiter un nombre d'évidements 180 strictement supérieur à deux, par exemple égal à trois.

En variante non représentée, les parties 16a et 16b de la gaine 16 peuvent être vissées l'une à l'autre ou liés par un mécanisme de verrouillage rotatif. Par exemple, le mécanisme peut comprendre un pion guidé dans une rainure curviligne ou coudée. Ce type de mécanisme est couramment appelée mécanisme de verrouillage à baïonnette. Dans tous les cas, les parties 16a et 16b sont détachables l'une de l'autre par un mouvement de rotation relatif entre les deux parties.

En variante non représentée, le joint de piston 6 n'est pas attaché à la tige 4, c'est-à-dire que la tige 4 est en appui simple contre le joint 6. Le joint 6 n'est alors lié à la tige 4 que dans un sens de déplacement.

Selon une autre variante non représentée, le container 100 délimite une ouverture ou une partie transparente pour la visualisation des seringues 1 de l'extérieur.

Les caractéristiques des variantes et modes de réalisation envisagés ci-dessus peuvent être combinées entre elles pour générer de nouveaux modes de réalisation de l'invention.

## Revendications

1. Procédé de fabrication de seringues pré-remplies à aiguille collée comprenant des étapes consistant à :
a) monter un dispositif (D) de protection de l'aiguille sur des corps de seringue (2), le dispositif comprenant un système de sécurité après usage (18, 20) et un protège-aiguille (12),
b) disposer les corps de seringue (2), équipés du dispositif de protection, dans des logements (O204) prévus dans un support (200),
c) placer le support (200) dans un container standard (100), fermer le container et stériliser le container pour le transport,
d) dans une ambiance stérile, sortir le support du container,
e) remplir chaque corps de seringue avec un principe actif (P),
**caractérisé en ce que** le support (200) est un support standard et **en ce que** le procédé comprend, en outre, des étapes consistant à :
f) insérer un piston (6) dans chaque seringue,
g) sortir les seringues (1) de leur support pour l'inspection, l'assemblage d'une tige de piston (4) standard et l'étiquetage, et à
h) emballer individuellement chaque seringue (1) avec un emballage primaire (300) et un emballage secondaire (400).

2. Procédé selon la revendication 1, **caractérisé en ce que**, à l'étape c), le support (200) est placé dans le container (100) de manière que les seringues (1) ne touchent pas le fond (102) du container (100).

3. Procédé selon la revendication 1 ou 2, **caractérisé en ce que** le dispositif (D) monté sur chaque corps de seringue présente à son extrémité distale un chanfrein annulaire (18.1) qui coopère sans accrocs avec un logement correspondant (O204) du support lors des étapes b) et g).

4. Procédé selon l'une des revendications 1 à 3, **caractérisé en ce que**, à l'étape g), la tige de piston (4) est vissée sur le piston (6).

5. Procédé selon l'une des revendications 1 à 4, **caractérisé en ce que** l'emballage primaire utilisé à l'étape g) est un emballage coque transparent (300).

6. Procédé selon l'une des revendications 1 à 5, **caractérisé en ce que** l'emballage secondaire utilisé à l'étape g) est une boite en carton (400).

7. Procédé selon l'une des revendications 1 à 6, **caractérisé en ce que** chaque dispositif comprend des moyens (166) de fixation élastique sur un corps de seringue correspondant, qui se fixent, lors de l'étape a), par rapprochement avec le corps de seringue.

8. Procédé selon la revendication 7, **caractérisé en ce que** les moyens de fixation incluent des pattes élastiques (166) qui coopèrent, lors de l'étape a), avec une partie d'extrémité (8) du corps de seringue, cette partie d'extrémité étant conformée pour bloquer le dégagement des pattes une fois le dispositif (D) monté.

9. Procédé selon l'une des revendications 1 à 8, **caractérisé en ce que** les étapes d) à f) sont automatisées.

10. Procédé selon l'une des revendications 1 à 9, **caractérisé en ce que** les logements (O204) du support standard (200) ont un diamètre de 9,3 mm
